(19) Europäisches Patentamt
European Patent Office
Office européen des brevets

(11) EP 0 812 596 A1

(12) **EUROPÄISCHE PATENTANMELDUNG**

(43) Veröffentlichungstag:
17.12.1997 Patentblatt 1997/51

(51) Int. Cl.$^6$: **A61M 5/168**, F16K 7/17,
G05D 7/01

(21) Anmeldenummer: 97109268.9

(22) Anmeldetag: 07.06.1997

(84) Benannte Vertragsstaaten:
AT BE CH DE DK ES FI FR GB GR IE IT LI LU MC NL PT SE

(30) Priorität: 14.06.1996 DE 29610419 U

(71) Anmelder: Filtertek, S.A.
F-60128 Plailly (FR)

(72) Erfinder:
**Myers, Jan Willem Marinus, Ing.**
5913 TP Venlo (NL)

(74) Vertreter: **Brose, D. Karl et al**
**Patentanwälte Brose & Brose**
**Postfach 11 64**
**82301 Starnberg (DE)**

(54) **Schwerkraft-Infusionsvorrichtung für medizinische Infusionen**

(57)    Die Erfindung betrifft eine Schwerkraft-Infusionsvorrichtung für medizinische Infusionen mit einer Tropfkammer (4), welche mittels eines Hohldorns (2) an eine die Infusionsflüssigkeit enthaltende Flasche (1) anschließbar ist. Eine flexible Leitung (6) ist an einem Ende (8) an die Tropfkammer (4) angeschlossen, und weist an ihrem anderen Ende (10) eine Kupplungseinrichtung (12) für die Infusionsnadel auf. Zwischen der Tropfkammer (4) und der Kupplungseinrichtung (12) ist in der flexiblen Leitung (6) eine Druckausgleichseinrichtung (16) vorgesehen, die mindestens einen Eingang (18) und mindstens einen Ausgang (20) für die Infusionsflüssigkeit aufweist und die auf Änderungen des hydrostatischen Drucks am Eingang (18) ansprechend, die gewählte Strömungsmenge am Ausgang konstant hält. Die Druckausgleichseinrichtung (16) weist eine Ausgleichskammer (22) auf, welche mit dem Eingang (18) und dem Ausgang (20) versehen ist. Zwischen dem Eingang (18) und dem Ausgang (20) ist eine dünne Membran im wesentlichen über den gesamten Querschnitt des Gehäuses (26) eingespannt, wobei eine seitliche Bypassöffnung (32) vorgesehen ist, welche die Oberseite (34) der Membran (24) mit deren Unterseite (36) verbindet. Die Membran (24) ändert bei Änderungen des auf der Seite des Eingangs (18) herrschenden Drucks den Strömungsweg derart, daß die Strömungsmenge auf der Seite des Ausgangs (20) konstant bleibt.

Fig. 3

EP 0 812 596 A1

Printed by Xerox (UK) Business Services
2.15.4/3.4

**Beschreibung**

Die Erfindung betrifft eine Schwerkraft-Infusionsvorrichtung für medizinische Infusionen mit einer Tropfkammer, welche mittels eines Hohldorns an eine die Infusionsflüssigkeit enthaltende Flasche anschließbar ist, wobei eine flexible Leitung an einem Ende an die Tropfkammer angeschlossen ist und an ihrem anderen Ende eine Kupplungseinrichtung für eine Infusionsnadel oder -katheter aufweist, und wobei zwischen der Tropfkammer und der Kupplungseinrichtung eine Vorrichtung zum Steuern der Strömungsmenge der Infusionsflüssigkeit vorgesehen ist.

In der Medizin gibt es zwei grundsätzliche Arten Patienten, durch Infusionstherapie zu behandeln.

Bei dem ersten Fall handelt es sich um die sogenannte Schwerkraftinfusion und beim zweiten Fall um Infusionen mittels einer motorisch angetriebenen Pumpe. In beiden Fällen kommt die Infusionsflüssigkeit aus einem Behälter, bei welchem es sich entweder um eine Flasche oder um eine gefüllte Spritze handelt. Das auf Schwerkraft beruhende Prinzip der Infusion ist das billigste, weist jedoch den Nachteil auf, daß es ziemlich ungenau ist, um beispielsweise Medikamente mittels Infusionen zu verabreichen. In diesem Zusammenhang ist es unverzichtbar, daß das Krankenhauspersonal mehrfach täglich die verschriebene Dosis per Infusionsdauer neu einstellt. Daher werden für genaue Dosierungen hauptsächlich die motorisch angetriebenen Systeme verwendet, welche doch vergleichsweise teuer sind.

Eine übliche Schwerkraft-Infusionsvorrichtung der eingangs genannten Art besteht üblicherweise aus den oben erwähnten genormten Bestandteilen, nämlich einer Tropfkammer, welche einen entlüfteten oder nicht entlüfteten Hohldorn oder Hohlnadel aufweist, mittels derer der sterile Verschluß des die Infusionsflüssigkeit enthaltenen Behälters durchstoßen wird, um dadurch die Verbindung gleichzeitig beim Öffnen des Behälters in steriler Weise herzustellen. Eine flexible Leitung verbindet die Tropfkammer mit dem distalen Ende, an welchem eine Kupplungseinrichtung zum Anschluß an eine Infusionsnadel- oder katheter vorgesehen ist. Bei der Kupplungseinrichtung handelt es sich üblicherweise um einen männlicher Luerlock-Anschluß, mittels dessen ein Langzeitkatheter, der mit dem Patienten verbunden ist, angeschlossen werden kann. Zwischen der Tropfkammer und letztlich dem Patienten ist noch eine Vorrichtung zum Steuern der Strömungsmenge der Infusionsflüssigkeit vorgesehen, mittels derer die Dosierung der Infusion verstellt werden kann. Bei einer derartigen Steuervorrichtung handelt es sich üblicherweise entweder um eine sogenannte Rollenklemme oder spezielle, als Einweggeräte ausgebildete Steuerventile, um ein noch genaueres Einstellen der Strömungsmengen zu ermöglichen.

Bei auf motorisch angetriebenen Pumpen basierenden Infusionssystemen kann man noch Kolbenpumpen und peristaltische Pumpen unterscheiden, wobei diese Pumpensysteme mit dem Patienten ebenfalls über flexible Leitungen verbunden werden.

Schwerkraft-Infusionsvorrichtungen sind einfach und billig in der Anwendung, weisen jedoch den Nachteil einer großen Ungenauigkeit auf. Infusionspumpen sind hier sehr viel genauer als die auf Schwerkraft beruhenden Infusionsvorrichtungen, sind jedoch ausgesprochen teuer. Kolbenpumpen werden in diesem Zusammenhang hauptsächlich für kleine Volumina verwendet. Es sind ferner erhebliche Verbesserungen hinsichtlich der Genauigkeit bekannt geworden, bei welchen in Kombination mit Schwerkraft-Infusionsvorrichtungen elektronische Tropfenzählsysteme verwendet werden, wobei derartige Systeme ebenfalls ausgesprochen teuer sind.

Aus der DE 27 13 618 C2 ist noch ein Durchflußsteuergerät, beispielsweise für die intravenöse Eingabe von Flüssigkeiten bekannt. Bei diesem bekannten Gerät handelt es sich um die Kombination eines Rückschlagventils mit einem Druckregler, der den Druck konstant halten soll. Der hiernach bekannte Druckregler teilt hierbei die Strömung in zwei getrennte Teilströme auf, wobei einer der Teilströme als Steuerdruck für die Membran des Druckreglers genutzt wird. Entsprechend ist dieses bekannte Durchflußsteuergerät vergleichsweise komplex aufgebaut.

Ein ebenfalls vergleichsweise kompliziert aufgebautes Druchflußsteuergerät für Infusionszwecke ist ferner aus der US-PS 4,343,305 bekannt. Bei diesem bekannten Gerät erfolgt die Einstellung der Strömungsmenge innerhalb des gleichen Gerätes, welches auch einen Druckregler enthält. Der Druckregler, welcher als Druckausgleichseinrichtung ausgebildet ist, um den Druck bei eingestellter konstanter Strömungsmenge konstant zu halten, enthält eine freiliegende Membran als Steuerglied, was im praktischen Einsatz zu erheblichen Ungenauigkeiten führen kann.

Der Erfindung liegt daher die Aufgabe zugrunde, eine Schwerkraft-Infusionsvorrichtung der eingangs genannten Art dahingehend zu verbessern, daß mit ausgesprochen einfachen und kostengünstigen Mitteln eine hohe Genauigkeit erreichbar wird. In diesem Zusammenhang ist darauf hinzuweisen, daß die Ungenauigkeit bekannter Schwerkraft-Infusionsvorrichtungen hauptsächlich dadurch verursacht werden, daß der hydrostatische Druck sich beim Entleeren des die Infusionsflüssigkeit enthaltenden Behälters oder Flasche ändert, oder daß sich bei Verwendung von Rollenklemmen durch Kaltfließen des Kunststoffs, aus welchem derartige Klemmen hergestellt werden, unbeabsichtigt der eingestellte Strömungsquerschnitt der Leitung ändert.

Bei einer Schwerkraft-Infusionsvorrichtung der oben genannten Art wird die der Erfindung zugrundeliegende Aufgabe im wesentlichen dadurch gelöst, daß in der flexiblen Leitung eine Durckausgleichseinrichtung vorgesehen ist, welche mindestens einen Eingang und mindestens einen Ausgang für die Infusionsflüssigkeit aufweist, und daß die Druckausgleichseinrichtung auf Änderungen des hydrostatischen Drucks am Eingang ansprechend die gewählte Strömungsmenge am Ausgang konstant hält, daß die Durckausgleichseinrichtung eine Ausgleichskammer enthält, welche mit dem Eingang und dem Ausgang versehen ist, daß zwischen dem Eingang und dem Ausgang eine dünne flexible Membran vorgesehen ist, die bei Änderungen des auf der Seite des Eingangs herrschenden hydrostatischen Drucks den Strömungsweg derart ändert, daß die Strömungsmenge auf der Seite des Ausgangs konstant bleibt, daß die Membran im wesentlichen über den gesamten Querschnitt des Gehäuses eingespannt ist, und daß eine seitliche Bypass-Öffnung vorgesehen ist, welche die Oberseite der Membran mit deren Unterseite verbindet.

Auf diese Weise wird durch die Druckausgleichseinrichtung sowohl der abfallende hydrostatische Druck beim Leeren des Infusionsbehälters als auch die Änderung der Strömungsöffnung in der Rollenklemme ausgeglichen, indem die Membran selbsttätig den inneren Strömungsweg im Sinne einer Konstanthaltung der Strömungsmenge entsprechend ändert. Diese Änderung steht in direkter Beziehung zum Abfallen des hydrostatischen Drucks beim Leeren des Behälters oder zu Änderungen im Druckabfall, wenn sich die Öffnung in der in der Rollenklemme zusammengedrückten flexiblen Leitung aufgrund des Kaltfließens des Kunststoffs ändert. Im ersten Falle wird durch die Membrane eine innere Steueröffnung im größeren Querschnitt freigegeben, wenn der hydrostatische Druck abfällt, und im zweiten Falle wird die innere Öffnung mehr oder weniger geschlossen, wenn der Druckabfall an der Rollenklemme zunimmt oder abnimmt. Selbstverständlich korrigiert auch die Membrane, wenn beide Phänomene gleichzeitig auftreten.

Bei einer bevorzugten Ausführungsform nach der Erfindung ist der Eingang an einen von der Tropfkammer ausgehenden ersten flexiblen Leitungsabschnitt und der Ausgang an einen zur Kupplungseinrichtung führenden flexiblen zweiten Leitungsabschnitt angeschlossen, wobei die Steuervorrichtung in dem zweiten Leitungsabschnitt angeordnet ist. Hiermit ist sichergestellt, daß die Gesamtmenge der zu verabreichenden Infusion durch die Durckausgleichseinrichtung geleitet wird.

Im einzelnen ist es bevorzugt, daß der Eingang und der Ausgang als koaxiale, an der Ober- bzw. Unterseite eines zylindrischen, die Ausgleichskammer bildenden Gehäuses angeordnete Anschlußstutzen ausgebildet sind. Hierdurch läßt sich die Druckausgleichseinrichtung einfach in bereits vorhandene Infusionsvorrichtungen einbauen, indem die vorhandene flexible Leitung durchtrennt wird und die beiden Leitungsabschnitte mittels der Anschlußstutzen wieder verbunden werden.

Im einzelnen ist es in diesem Zusammenhang vorteilhaft, daß koaxial zu dem den Ausgang bildenden Anschlußstutzen ein in die Ausgleichskammer im Gehäuse vorstehender Stutzen vorgesehen ist, welche eine der Unterseite der Membran gegenüberliegende Ventilfläche aufweist.

Ferner ist es bevorzugt, daß im Eingang der Ausgleichskammer eine Drosselstelle vorgesehen ist.

Nach einer weiteren vorteilhaften Ausgestaltung der Erfindung soll der Ausgang der Ausgleichskammer eine trichterförmige Drosselstelle aufweisen.

Bei einer besonders bevorzugten Weiterbildung nach der Erfindung ist die Durckausgleichseinrichtung als Einwege- oder Wegwerfteil ausgebildet, wobei das Gehäuse der Druckausgleichseinrichtung aus Kunststoff besteht, und daß das Gehäuse aus einem Deckelteil und einem Bodenteil besteht. In vorteilhafter Weise ist der Deckelteil einstückig mit dem Anschlußstutzen und der Bypass-Öffnung und der Bodenteil einstückig mit dem Anschlußstutzen und dem die Ventilfläche aufweisenden Stutzen ausgebildet.

Bei einer vorteilhaften Weiterbildung dieser Ausführungsform ist vorgesehen, daß die Membran zwischen Deckelteil und Bodenteil mittels geeigneter einstückiger Formationen eingespannt ist. Bei dieser Ausführungsform besteht der erreichte Vorteil darin, daß die gesamte Druckausgleichseinrichtung lediglich aus drei Teilen, nämlich dem Bodenteil, dem Deckelteil und der Membran besteht, welche kostengünstig herstellbar und zusammenbaubar sind, was ja Voraussetzung für die Ausbildung als Einwege- oder Wegwerfteil ist.

Da es bei Schwerkraft-Infusionsvorrichtungen der erfindungsgemäßen Gattung vorkommen kann, daß unter bestimmten Umständen zusätzliche Rückschlagventile erforderlich werden, ist bei einer besonders bevorzugten Ausführungsform nach der Erfindung auf der Seite des Eingangs ein Ventilsitz vorgesehen, welcher mit der Membran 24 ein Rückschlagventil bildet. Dieses Rückschlagventil wird nur dann wirksam, wenn unter bestimmten Umständen der Gegendruck in der Infusionsleitung höher wird als der gesamte hydrostatische Druck in der an dem Patienten angeschlossenen Leitung, sodaß in diesem Falle die flexible Membran umgehend die Leitung zu der Flasche abschaltet und somit keine Möglichkeit besteht, daß die Infusionsleitung beispielsweise mit Blut vom Patienten kontaminiert wird, oder im Falle einer Okklusion mit einem Medikament welches beispielsweise von einer Infusionspumpe kommt.

Diese Ausführungsform ist bevorzugt derart ausgestaltet, daß der Ventilsitz als von dem Deckelteil in Richtung der Membran vorstehender, den Eingang umgebender Ring, ausgebildet ist, welcher ferner bevorzugt einstückig mit dem Deckelteil und mit einem V-förmigen Querschnitt ausgebildet ist.

Weiterer Gegenstand der Erfindung ist die Druckausgleichseinrichtung als solche, welche ein oder mehrere der oben erwähnten Konstruktionsmerkmale aufweist.

Im folgenden wird die Erfindung anhand einer in den Zeichnungen beispielhaft veranschaulichten Ausführungsform näher erläutert. Es zeigt:

**FIGUR 1** eine schematische Seitenansicht einer Schwerkraft-Infusionsvorrichtung nach der Erfindung

**FIGUR 2** eine schematische seitliche Schnittansicht der erfindungsgemäß vorgesehenen Druckausgleichseinrichtung im vergrößerten Maßstab;

**FIGUR 3** eine abgewandelte Ausführungsform der Druckausgleichseinrichtung, welche gleichzeitig als Rückschlagventil dient;

**FIGUR 4** eine Unteransicht des Deckelteils der Ausführungsform gem. Fig. 3.

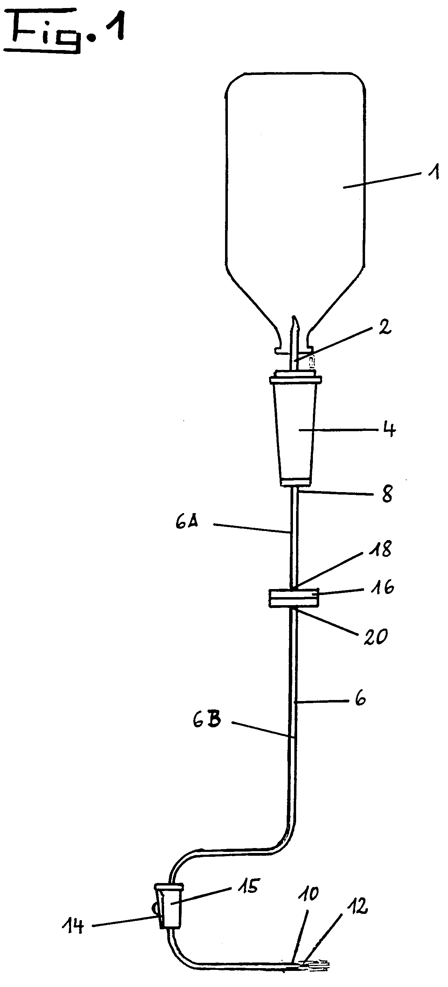

Wie in Figur 1 gezeigt, besteht eine Schwerkraft-Infusionsvorrichtung für medizinische Infusionen aus der die Infusionsflüssigkeit enthaltenen Flasche 1, welche üblicherweise erhöht aufgehängt wird. Über einen Hohldorn oder Nadel 2, der Bestandteil der üblichen Tropfkammer 4 ist, ist eine flexible Leitung 6 an die Flasche 1 angeschlossen. Die flexible Leitung 6 ist mit ihrem ersten Ende 8 an das Unterende der Tropfkammer 4 in üblicher Weise angeschlossen, während ihr zweites Ende 10 eine allgemein mit 12 bezeichnete Kupplungseinrichtung aufweist, die für den Anschluß an eine mit dem Patienten verbundene Infusionsnadel oder -katheter dient. Bei der Kupplungseinrichtung 12 handelt es sich üblicherweise um den männlichen Teil eines Luerlock-Anschlusses.

Zwischen der Tropfkammer 4 und der Kupplungseinrichtung 12 ist ferner eine allgemein mit 14 bezeichnete Steuervorrichtung zum Steuern der Strömungsmenge der Infusionsflüssigkeiten zum Patienten vorgesehen, bei welcher es sich üblicherweise um eine Rollenklemme 15 handelt.

Erfindungsgemäß ist an geeigneter Stelle in der flexiblen Leitung 6 zwischen der Tropfkammer 4 und der Steuervorrichtung 14 eine Durckausgleichseinrichtung 16 vorgesehen, welche einen Eingang 18 und einen Ausgang 20 für die Infusionsflüssigkeit aufweist. Die Durckausgleichseinrichtung 16 ist hierbei derart ausgebildet, daß sie, wie weiter unten noch näher erläutert wird, auf Änderungen des hydrostatischen Drucks am Eingang 18 ansprechend die gewählte Strömungsmenge am Ausgang 20 konstant hält.

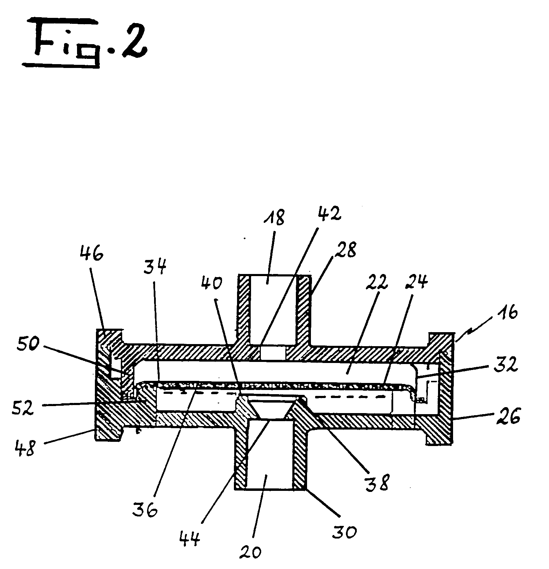

Wie insbesondere aus Figur 2 ersichtlich, weist die Druckausgleichseinrichtung 16 eine Ausgleichskammer 22 auf, die mit dem Eingang 18 und dem Ausgang 20 versehen ist. Zwischen dem Eingang 18 und dem Ausgang 20 ist eine dünne flexible Membran 24 vorgesehen, die durch die Flüssigkeitssäule oberhalb des Eingangs 18 belastet ist. Die Membran 24 wird durch Änderungen des auf der Seite des Eingangs 18 herrschenden hydrostatischen Drucks derart verformt, daß die Strömungsmenge auf der Seite des Ausgangs 20 konstant gehalten wird.

Der Eingang 18 ist an einen von der Tropfkammer ausgehenden ersten Leitungsabschnitt 6A der flexiblen Leitung und der Ausgang 20 an einen zur Kupplungseinrichtung 12 führenden zweiten Leitungsabschnitt 6B der flexiblen Leitung 6 angeschlossen, wobei die Steuervorrichtung 14 in dem zweiten Leitungsabschnitt 6B angeordnet ist (sh. Figur 1).

Wie aus Figur 2 ersichtlich, sind der Eingang 18 und der Ausgang 20 als koaxiale, auf der Oberseite bzw. Unterseite eines zylindrischen Gehäuses 26 angeordnete Anschlußstutzen 28 und 30 ausgebildet, wobei das Gehäuse 26 die Ausgleichskammer 22 umschließt. Auf die Anschlußstutzen 28 und 30 lassen sich die entsprechenden Enden der Leitungsabschnitte 6A bzw. 6B aufschieben.

Die Membran 24 überspannt im wesentlichen den Gesamtquerschnitt des Gehäuses 26, wobei eine seitliche Bypass-Öffnung 32 vorgesehen ist, welche die Oberseite 34 der Membran 24 mit deren Unterseite 36 verbindet. Die durch den Eingang 18 einströmende Infusionsflüssigkeit gelangt somit von der Oberseite 34 der Membran 24 durch die Bypass-Öffnung 32 zur Unterseite 36 der Membran 24 und von dort zum Ausgang 20 und letztlich zum Patienten.

Koaxial zu dem den Ausgang 20 bildenden Anschlußstutzen 30 ist ein in die Ausgleichskammer 22 des Gehäuses 26 vorstehender Stutzen 38 vorgesehen, welcher eine der Unterseite 36 der Membran 24 gegenüberliegende Ventilfläche 40 aufweist. Die Ventilfläche 40 bildet zusammen mit der gegenüberliegenden Unterseite 36 der Membran 24 die durch den hydrostatischen Druck gesteuerte Öffnung, die, wie weiter unten noch näher erläutert wird, gewährleistet, daß auf der stromabwärts liegenden Seite konstante Strömungsverhältnisse herrschen, obwohl sich auf der Eingangsseite der hydrostatische Druck ändert.

Im Eingang 18 der Ausgleichskammer 22 ist eine Drosselstelle 42 vorgesehen. Eine weitere trichterförmig ausgebildete Drosslstelle 44 ist im Ausgang 20 der Ausgleichskammer ausgebildet.

Wie gezeigt, ist gemäß Figur 2 die Druckausgleichseinrichtung 16 bevorzugt als Wegwerf- oder Einwegeteil ausgebildet. Zu diesem Zweck besteht das Gehäuse 26 der Druckausgleichseinrichtung 16 aus Kunststoff, und ist durch einen Deckelteil 46 und einen Bodenteil 48 gebildet, die miteinander verbunden sind. Der Deckelteil 46 ist einstückig mit dem Anschlußstutzen 28 und der Bypass-Öffnung 32 ausgebildet, wobei der Bodenteil 48 einstückig mit dem Anschlußstutzen 30 und dem die Ventilfläche 40 aufweisenden Stutzen 38 ausgebildet ist. Die Membran 24 ist zwischen dem Deckelteil 46 und dem Bodenteil 48 mittels geeigneter, ebenfalls einstückig ausgebildeter Formationen 50 und 52 eingespannt gehalten.

Beim praktischen Einsatz der beschriebenen Ausführungsform fließt die Infusionsflüssigkeit aus der Flasche 1 über die Tropfkammer 4 und den ersten Leitungsabschnitt 6A am Eingang 18 in die Durckausgleichseinrichtung 16 und durch die innere Bypass-Öffnung 32 zum Ausgang 20. Der Ausgang 20 ist mit dem Patienten über den zweiten Leitungsabschnitt 6B der flexiblen Leitung 6 verbunden. Nach der Entlüftung oder nach Füllen der gesamten Infusionsvorrichtung mit Flüssigkeit lenkt die Flüssigkeitssäule oberhalb der dünnen Membran 24 die dünne Gummimembrane aus und verringert somit den Strömungsquerschnitt zwischen der Unterseite 36 der Membran 24 und der Ventilfläche 40. Die Verringerung dieses Strömungsquerschnitts führt zu einer geringeren Strömungsmenge. Wenn das Flüssigkeitsniveau in dem System absinkt, läßt auch die Auslenkung der Membrane nach, was in anderen Worten bedeutet, daß der Strömungsquerschnitt zwischen der Unterseite 36 der Membran 24 und der gegenüberliegenden Ventilfläche 40 größer wird. Folglich kann mehr Flüssigkeit durch diesen Strömungsquerschnitt fließen. Obwohl somit der hydrostatische Druck abfällt, bleibt die Flüssigkeitsmenge konstant. Die Beziehung zwischen Strömungsmenge, Druckabfall und Öffnungsfläche ist leicht aus der Poiseuille'schen Gleichung ersichtlich:

$$Q = \frac{C \times p \times d^4}{l}$$

wobei

Q die Stömungsmenge,
C die Konstante
p die Druckänderung
d der Öffnungsquerschnitt und
l die Länge der Öffnung

bedeutet.

Die Gleichung Zeigt, daß die Strömungsmenge durch den Druckabfall aufgrund des Entleerens der Infusionsflasche 1 beeinflußt wird. Da die flexible Membran 24 durch den hydrostatischen Druck verformt wird, verringert sie den Strömungsquerschnitt und läßt einen schmalen Spalt frei, durch welchen die Flüssigkeit den Ausgang 20 der Druckausgleichseinrichtung 16 findet. Wenn der hydrostatische Druck im Verlaufe der Infusion abnimmt, nimmt auch die durch diesen auf die flexible Membran 16 ausgeübte Kraft ab. Dadurch kehrt die verformte Membran 16 langsam in ihre gerade Lage zurück und gibt den Strömungsquerschnitt mehr und mehr frei, so daß mehr Flüssigkeit hindurchströmen kann. Folglich wird der Abfall des hydrostatischen Drucks selbsttätig ausgeglichen.

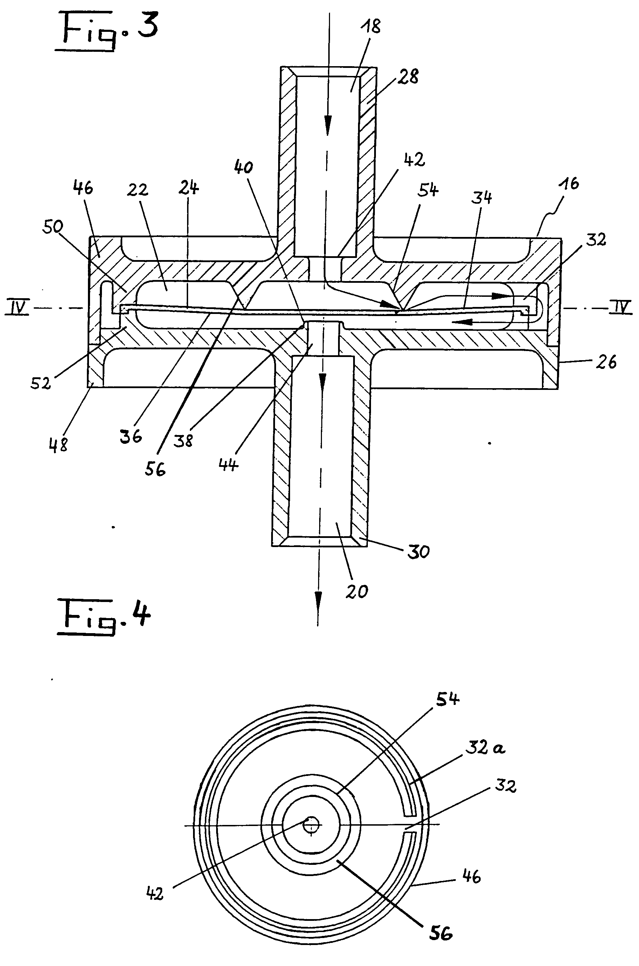

In den Figuren 3 und 4 ist eine abgewandelte besonders bevorzugte Ausführungsform nach der Erfindung gezeigt, wobei für gleiche oder gleichwirkende Teile und Einzelheiten die gleichen Bezugszeichen wie in den Figuren 1 und 2 verwendet wurden.

Wie gezeigt ist bei dieser Ausführungsform die Druckausgleichseinrichtung 16 mit einem Rückschlagventil kombiniert, indem in der Ausgleichskammer 22, das heißt, auf der Seite des Eingangs 18 ein der Membran 24 gegenüberliegender Ventilsitz 54 vorgesehen ist. Der Ventilsitz 54 ist als den Eingang 18 umgebender Ring 56 mit einem V-förmigen Querschnitt ausgebildet, wobei der Ring 56 einstückig mit dem Deckelteil 46 ausgeformt ist.

Aus der den Deckelteil 46 in einer Unteransicht zeigenden Darstellung gem. Fig. 4 ist ferner ersichtlich, daß wie auch bei der Ausführungsform gem. Fig. 2, die Bypassöffnung 32 mit einem um den Deckelteil 46 umlaufenden Bypasskanal 32 a verbunden ist.

Sämtliche der aus der Beschreibung, den Ansprüchen und Zeichnungen hervorgehenden Merkmale und Vorteile der Erfindung, einschließlich konstruktiver Einzelheiten und räumlicher Anordnungen, können sowohl für sich als auch in beliebiger Kombination erfindungswesentlich sein.

## BEZUGSZEICHENLISTE

| | |
|---|---|
| 1 | = Flasche |
| 2 | = Hohldorn oder Nadel |
| 4 | = Tropfkammer |
| 6 | = flexible Leitung |
| 6A | = erster Leitungsabschnitt |
| 6B | = zweiter Leitungsabschnitt |
| 8 | = erstes Ende von 6 |
| 10 | = zweites Ende von 6 |
| 12 | = Kupplungseinrichtung |
| 14 | = Steuervorrichtung |
| 15 | = Rollenklemme |
| 16 | = Druckausgleichseinrichtung |
| 18 | = Eingang von 16 |
| 20 | = Ausgang von 16 |
| 22 | = Ausgleichskammer |
| 24 | = Membrane |
| 26 | = Gehäuse |
| 28 | = Anschlußstutzen |
| 30 | = Anschlußstutzen |
| 32 | = Bypass-Öffnung |
| 34 | = Oberseite von 24 |
| 36 | = Unterseite von 24 |
| 38 | = Stutzen |
| 40 | = Ventilfläche |
| 42 | = Drosselstelle |
| 44 | = Drosselstelle |
| 46 | = Deckelteil |
| 48 | = Bodenteil |
| 50 | = Formationen |
| 52 | = Formationen |
| 54 | = Ventilsitz |
| 56 | = Ring |

## Patentansprüche

1. Schwerkraft-Infusionsvorrichtung für medizinische Infusionen, mit einer Tropfkammer, welche mittels eines Hohldorns an eine, die Infusionsflüssigkeit enthaltende Flasche anschließbar ist, wobei eine flexible Leitung an einem Ende an die Tropfkammer angeschlossen ist und an ihrem anderen Ende eine Kupplungseinrichtung für einen Infusionsnadel- oder -katheter aufweist, und wobei zwischen der Tropfkammer und der Kupplungseinrichtung eine Vorrichtung zum Steuern der Strömungsmenge der Infusionsflüssigkeit vorgesehen ist, **dadurch gekennzeichnet**, daß in der flexiblen Leitung (6) eine Druckausgleichseinrichtung (16) vorgesehen ist, welche mindestens einen Eingang (18) und mindestens einen Ausgang (20) für die Infusionsflüssigkeit aufweist, und welche auf Änderungen des hydrostatischen Drucks am Eingang (18) ansprechend die gewählte Strömungsmenge am Ausgang (20) konstant hält, daß die Druckausgleichseinrichtung (16) eine Ausgleichskammer (22) aufweist, welche mit dem Eingang (18) und dem Ausgang (20) versehen ist, daß zwischen dem Eingang (18) und dem Ausgang (20) eine dünne flexible Membran (24) vorgesehen ist, welche bei Änderungen des auf der Seite des Eingangs (18) herrschenden hydrostatischen Drucks den Strömungsweg derart ändert, daß die Strömungsmenge auf der Seite des Ausgangs (20) konstant bleibt, daß die Membran (24) im wesentlichen über den gesamten Querschnitt des Gehäuses (26) eingespannt ist, und daß eine seitliche Bypass-Öffnung (32) vorgesehen ist, welche die Oberseite (34) der Membran (24) mit deren Unterseite (36) verbindet.

2. Schwerkraft-Infusionsvorrichtung nach Anspruch 1, **dadurch gekennzeichnet**, daß der Eingang (18) an einen, von der Tropfkammer (4) ausgehenden ersten flexiblen Leitungsabschnitt (6A) und der Ausgang (20) an einen zur Kupplungseinrichtung (12) führenden flexiblen zweiten Leitungsabschnitt (6b) angeschlossen ist, wobei die Steuervorrichtung (14) in dem zweiten Leitungsabschnitt (6B) angeordnet ist.

3. Schwerkraft-Infusionsvorrichtung nach Anspruch 1 oder 2, **dadurch gekennzeichnet**, daß der Eingang (18) und der Ausgang (20) als koaxial an der Ober- bzw. Unterseite eines zylindrischen, die Ausgleichskammer (22) bildenden Gehäuses (26) angeordnete Anschlußstutzen (28, 30) ausgebildet sind.

4. Schwerkraft-Infusionsvorrichtung nach einem der vorstehenden Ansprüche, **dadurch gekennzeichnet**, daß koaxial zu dem den Ausgang (20) bildenden Anschlußstutzen (30) ein in die Ausgleichskammer (22) im Gehäuse (26) vorstehender Stutzen (38) vorgesehen ist, welcher eine der Unterseite (36) der Membran(24) gegenüberliegende Ventilfläche (40) aufweist.

5. Schwerkraft-Infusionsvorrichtung nach einem der vorstehenden Ansprüche, **dadurch gekennzeichnet**, daß im Eingang (18) der Ausgleichskammer (22) eine Drosselstelle (42) vorgesehen ist.

6. Schwerkraft-Infusionsvorrichtung nach einem der vorstehenden Ansprüche, **dadurch gekennzeichnet**, daß der Ausgang (20) der Ausgleichskammer (22) eine trichterförmige Drosselstelle (44) aufweist.

7. Schwerkraft-Infusionsvorrichtung nach einem der vorstehenden Ansprüche, **dadurch gekennzeichnet**, daß die Druckausgleichseinrichtung (16) als Einwege- oder Wegwerfteil ausgebildet ist, daß das Gehäuse (26) der Druckausgleichseinrichtung (16) aus Kunststoff besteht und daß das Gehäuse (26) aus einem Deckelteil (46) und einem Bodenteil (48) besteht.

8. Schwerkraft-Infusionsvorrichtung nach Anspruch 7, **dadurch gekennzeichnet**, daß der Deckelteil (46) einstückig mit dem Anschlußstutzen (28) und der Bypass-Öffnung (32) ausgebildet ist.

9. Schwerkraft-Infusionsvorrichtung nach Anspruch 7 oder 8, **dadurch gekennzeichnet**, daß der Bodenteil (48) einstückig mit dem Anschlußstutzen (30) und dem die Ventilfläche (40) aufweisenden Stutzen (38) ausgebildet ist.

10. Schwerkraft-Infusionsvorrichtung nach einem der Ansprüche 7 bis 9, **dadurch gekennzeichnet**, daß die Membran (24) zwischen dem Deckelteil (46) und dem Bodenteil (48) mittels geeigneter einstückiger Formationen (50, 52) eingespannt ist.

11. Schwerkraft-Infusionsvorrichtung nach einem der vorstehenden Ansprüche, **dadurch gekennzeichnet**, daß auf der Seite des Eingangs (18) ein Ventilsitz (54) vorgesehen ist, welcher mit der Membran (24) ein Rückschlagventil bildet.

12. Schwerkraft-Infusionsvorrichtung nach Anspruch 11, **dadurch gekennzeichnet,** daß der Ventilsitz (54) als von dem Deckelteil (46) in Richtung der Membran (24) vorstehender, den Eingang (18)umgebender Ring (56) ausgebildet ist.

13. Schwerkraft-Infusionsvorrichtung nach Anspruch 12, **dadurch gekennzeichnet,** daß der Ring (56) einstückig mit dem Deckelteil (46) und mit einem V-förmigen Querschnitt ausgebildet ist.

# Fig.1

# Fig.2

Fig. 3

Fig. 4

# EP 0 812 596 A1

**Europäisches Patentamt**

## EUROPÄISCHER RECHERCHENBERICHT

Nummer der Anmeldung

EP 97 10 9268

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A,D | US 4 343 305 A (BRON )<br>* Zusammenfassung *<br>* Spalte 1, Zeile 42 - Spalte 2, Zeile 10; Abbildungen 1-6 *<br>--- | 1-13 | A61M5/168<br>F16K7/17<br>G05D7/01 |
| A | US 3 779 274 A (KELLY )<br>--- | | |
| A | US 3 633 605 A (SMITH )<br>* Zusammenfassung *<br>----- | | |

**RECHERCHIERTE SACHGEBIETE (Int.Cl.6)**

A61M
F16K
G05D

Der vorliegende Recherchenbericht wurde für alle Patentansprüche erstellt

| Recherchenort | Abschlußdatum der Recherche | Prüfer |
|---|---|---|
| DEN HAAG | 9.September 1997 | Michels, N |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes Dokument

EPO FORM 1503 03.82 (P04C03)

10